# EUROPÄISCHE PATENTSCHRIFT

(11) **EP 1 186 593 B1**
(45) Veröffentlichungstag und Bekanntmachung des Hinweises auf die Patenterteilung: **02.11.2005**
(21) Anmeldenummer: 01118076.7
(22) Anmeldetag: 26.07.2001
(51) Int. Cl.: C07C 69/82, C07C 67/08, C07C 67/54

(54) **Verfahren zur Herstellung von Carbonsäureestern**
Process for the preparation of carboxylic esters
Procédé pour la préparation d'esters carboxyliques

(30) Priorität: 05.09.2000 DE 10043545
(43) Veröffentlichungstag der Anmeldung: 13.03.2002
(73) Patentinhaber: Oxeno Olefinchemie GmbH, 45764 Marl (DE)
(72) Erfinder: Gubisch, Dietmar, Dr., 45772 Marl (DE); Ernst, Uwe, 45770 Marl (DE); Büschken, Wilfried, Dr., 45721 Haltern (DE)

(56) Entgegenhaltungen:
- DE-A- 19 721 347
- GB-A- 1 372 854
- US-A- 2 130 014
- US-A- 2 462 601
- US-A- 5 648 517

## Beschreibung

Die Erfindung betrifft ein diskontinuierliches Verfahren zur Herstellung von Carbonsäureestern durch Umsetzung von di- oder mehrbasischen Carbonsäuren oder deren Anhydride mit Alkoholen.

Ester aus mehrbasischen Carbonsäuren, wie beispielsweise Phthalsäure, Adipinsäure, Sebacinsäure, Maleinsäure und Alkoholen finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Ganz gleich welche Art der Katalyse gewählt wird, es entsteht immer ein temperaturabhängiges Gleichgewicht zwischen den Einsatzstoffen (Carbonsäure und Alkohol) und den Produkten (Ester und Wasser). Um das Gleichgewicht zu Gunsten des Esters zu verschieben, wird bei vielen Veresterungen ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von zwei oder mehrbasigen Säuren ist in der Regel der eingesetzte Alkohol das Schleppmittel. Für viele Anwendungszwecke muss der so hergestellte Ester eine niedrige Säurezahl besitzen, d. h. die Umsetzung der Carbonsäure sollte praktisch quantitativ erfolgen. Anderenfalls wird die Ausbeute gemindert und die Säure muss, beispielsweise durch Neutralisation, abgetrennt werden. Dies ist aufwendig und kann zu Nebenprodukten führen, die entsorgt werden müssen. Um einen möglichst hohen Umsatz der Carbonsäure zu erhalten, werden Veresterungen in der Regel mit einem Alkoholüberschuss durchgeführt. Ein Alkoholüberschuss hat jedoch den Nachteil, dass bei niedrig siedendenden Alkoholen die Reaktionstemperatur bei Normaldruck so niedrig ist, dass die Reaktionsgeschwindigkeit für ein technisches Verfahren zu gering wird. Um diesem Effekt entgegenzuwirken, kann die Reaktion unter Druck ausgeführt werden, was höhere Apparatekosten zur Folge hat. Ein weiterer Nachteil besteht darin, dass mit zunehmenden Alkoholüberschuss die maximal mögliche Konzentration des Zielproduktes im Reaktionsgefäß sinkt und damit die Chargenausbeute abnimmt. Darüber hinaus muss der im Überschuss eingesetzte Alkohol vom Ester abgetrennt werden, was Zeit- und Energieaufwand bedingt.

Veresterungsreaktionen für die Weichmacherester Dioctylphthalat (DOP) und Diisononylphthalat (DINP) mit Titanorganyl-Katalyse sind gut untersuchte Reaktionen und z. B. in GB 2 045 767, DE 197 21 347 oder US 5 434 294 beschrieben.

Diese Verfahren beinhalten die folgende Schritte:
- Reaktion von einem Molekül Phthalsäureanhydrid mit einem Molekül Alkohol zum Halbester (Estercarbonsäure), autokatalytisch
- Zugabe von Titan-Katalysator, z. B. n-Butyltitanat
- Reaktion von einem Molekül Halbester mit einem Molekül Alkohol unter Wasserabspaltung zum Diphthalat
- Gleichzeitige Entfernung des Reaktionswassers durch Abdestillieren eines Alkohol/Wasser-Azeotrops
- Zerstörung des Katalysators durch Basenzugabe
- Abdestillieren des überschüssigen Alkohols
- Abfiltrieren der Katalysatorreste
- Destillative Reinigung des Phthalsäurediesters, z. B. Wasserdampfdestillation.

In GB 1,372,854 wird ein Verfahren zur Herstellung von Carbonsäureestern beschrieben, bei dem während der Reaktion eine azeotrope Destillation durchgeführt wird, mit der Wasser und Alkohol aus dem Reaktionsgemisch abgetrennt wird, wobei das Wasser aus dem Destillat mittels einer Dean-Starke Apparatur abgetrennt wird und der Alkohol zurückgeführt wird.

In US 5,648,517 wird ein ähnliches Verfahren zur Herstellung von Carbonsäureestern beschrieben, wobei das Destilllat durch ein Membran-Modul in Wasser und Alkohol aufgetrennt wird und der Alkohol in das Reaktionsgemisch zurückgeführt wird.

Diese Verfahren sind diskontinuierlich und im Hinblick auf die maximale Ausnutzung der Reaktoren, d. h. der Raum-Zeit-Ausbeute noch nicht optimiert.

Es bestand daher die Aufgabe, die Verfahren für die diskontinuierliche Herstellung von Estern zu verbessern, um höhere Raum-Zeit-Ausbeuten (kurze Reaktionszeiten, hohe Chargenausbeuten) zu ermöglichen.

Es wurde gefunden, dass die Raum-Zeit-Ausbeute eines diskontinuierlichen Veresterungsverfahren, bei dem das Reaktionswasser als Azeotrop mit dem im Überschuss eingesetzten Alkohol destillativ entfernt und der so entfernte Alkohol ganz oder teilweise wieder ersetzt wird, erhöht werden kann, wenn der Füllstand des Reaktors möglichst konstant gehalten wird, indem die abgetrennte Flüssigkeitsmenge, d. h. Alkohol und Wasser ganz wieder durch Alkohol ersetzt wird.

Gegenstand der vorliegenden Erfindung ist daher ein Verfahren zur Herstellung von Carbonsäureestern durch Reaktion von Di- oder Polycarbonsäuren oder deren Anhydride mit Alkoholen, wobei das Reaktionswasser durch azeotrope Destillation mit dem Alkohol entfernt wird, wobei die durch die azeotrope Destillation aus der Reaktion entfernte Flüssigkeitsmenge vollständig mit dem Alkohol wieder ergänzt wird.

Als Flüssigkeitsmenge wird im folgenden das durch azeotrope Destillation aus der Reaktion entfernte Flüssigkeitsvolumen, hauptsächlich bestehend aus Reaktionswasser und Alkohol, bezeichnet.

Es ist ein vollständiger Ersatz der entfernten Flüssigkeitsmenge bevorzugt. Dies kann z. B. durch eine standgeregelte Einspeisung von Alkohol in den Reaktor erfolgen.

Es kann auch erforderlich sein, mehr als die abdestillierte Flüssigkeitsmenge in den Reaktor zurückzuführen, d. h. neben der entfernten Alkoholmenge wird das Reaktionswasser ersetzt und darüber hinaus weiterer Alkohol zugegeben. In dieser Ausführungsform wird 110 bis 100 %, bevorzugt 105 - 100 % der entfernten Flüssigkeitsmenge durch Alkohol ersetzt.

Das erfindungsgemäße Verfahren hat den Vorteil, dass im Vergleich zu bekannten diskontinuierlichen Verfahren die Reaktionsgeschwindigkeit erhöht wird. Dadurch kann die Taktzeit verkürzt werden, wodurch eine höhere Raum-Zeit-Ausbeute erreicht wind.

Das erfindungsgemäße Verfahren ist prinzipiell auf alle Veresterungen, bei denen das Reaktionswasser mit einem Alkohol destillativ abgetrennt wird, anwendbar.

Im erfindungsgemäßen Verfahren werden als Säurekomponente Di- und Polycarbonsäuren sowie deren Anhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden. Die Säuren können aliphatisch, carbocyclisch, heterocyclisch, gesättigt oder ungesättigt sowie aromatisch sein. Aliphatische Carbonsäuren besitzen mindestens 4 C-Atome. Beispiele für aliphatische Carbonsäuren bzw. deren Anhydride sind: Maleinsäure, Fumarsäure, Maleinsäureanhydrid, Bernsteinsäure, Bernsteinsäureanhydrid, Adipinsäure, Korksäure, Trimethyladipinsäure, Azelainsäure, Decandisäure, Dodecandisäure, Brassylsäure. Beispiele für carbocyclische Verbindungen sind: Hexahydrophthalsäureanhydrid, Hexahydrophthalsäure, Cyclohexan-1.4-dicarbonsäure, Cyclohex-4-en-1.2-dicarbonsäure, Cyclohexen-1.2-dicarbonsäureanhydrid, 4-Methylcyclohexan-1.2-dicarbonsäure, 4-Methylcyclohexan-1.2-dicarbonsäureanhydrid, 4-Methylcyclohex-4-en-1.2-dicarbonsäure, 4-Methylcyclohex-4-en-1.2-dicarbonsäureanhydrid. Beispiele für aromatische Verbindungen sind: Phthalsäure, Phthalsäureanhydrid, Isophthalsäure, Therephthalsäure, Trimellitsäure, Trimesinsäure, Pyromellitsäure, Pyromellitsäureanhydrid oder Naphthalindicarbonsäuren.

Im erfindungsgemäßen Verfahren werden bevorzugt verzweigte oder lineare aliphatische Alkohole mit 4 bis 13 C-Atomen eingesetzt. Die Alkohole sind einwertig und können sekundär oder primär sein.
Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind beispielsweise n-Butanol, Isobutanol, n-Octanol (1), n-Octanol(2), 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägern eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-Festbett-Katalysatoren ausgeübt, wie beispielsweise der OCTOL-Process (Hydrocarbon Proess., Int. Ed. (1986) 65 (2. Sect. 1), Seite 31-33).

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess, mit anschließender Hydroformylierung und Hydrierung hergestellt werden.

Die erfindungsgemäße Veresterung kann autokatalysiert oder katalysiert durchgeführt werden. Als Veresterungskatalysatoren können Lewis- oder Bröndstedtsäuren oder metallorganische Stoffe, die nicht unbedingt als Säure wirken müssen, eingesetzt werden. Bevorzugte Veresterungskatalysatoren sind Alkoholate, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt.

Die Veresterung wird in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist ein Einsatzstoff bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, ist es ratsam, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Estercarbonsäuren (Halbestern), beispielsweise Phthalsäureanhydrid zum Phthalsäuremonoester. Daher ist ein Katalysator häufig erst nach dem ersten Reaktionsschritt erforderlich.

Der umzusetzende Alkohol, der als Schleppmittel dient, kann im stöchiometrischen Überschuss, bevorzugt 5 bis 50 %, besonders bevorzugt 10 bis 30 % der stöchiometrisch notwendigen Menge eingesetzt werden.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,3 Massen-%.

Die Reaktionstemperaturen liegen bei Verwendung von Titankatalysatoren zwischen 160 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei verminderten Druck durchgeführt. Beispielsweise wird bei der Umsetzung von Phthalsäureanhydrid mit einem Gemisch isomerer Nonanole in einem Temperaturbereich von 170 °C bis 250 °C im Druckbereich von 1 bar bis 10 mbar gearbeitet.

Die in die Reaktion erfindungsgemäß zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem Vorratsgefäß bereitstehenden Alkohol zu ersetzen.

In anderen Ausführungsformen der Erfindung wird die abgetrennte Flüssigkeit auf den Alkohol aufgearbeitet.

Während der Reaktion wird ein Alkohol-Wasser-Gemisch als Azeotrop aus der Reaktionsmischung abdestilliert. Die Brüden verlassen über eine kurze Kolonne (Einbauten oder Füllkörper; 1 bis 5, vorzugsweise 1 bis 3 theoretische Böden) das Reaktionsgefäß und werden kondensiert. Das Kondensat kann in eine wässrige und in eine alkoholische Phase getrennt werden; dies kann eine Kühlung erforderlich machen. Die wässrige Phase wird abgetrennt und kann, gegebenenfalls nach Aufarbeitung, verworfen oder als Strippwasser bei der Nachbehandlung des Esters verwendet werden.

Die alkoholische Phase, die nach Trennung des azeotropen Destillat anfällt, kann teilweise oder vollständig in das Reaktionsgefäß zurückgeführt werden. In der Praxis hat sich eine standgeregelte Füllstandskontrolle der Reaktion zur Zufuhr des Alkohols bewährt.

Es ist möglich, die durch die azeotrope Destillation entfernte Flüssigkeitsmenge vollständig oder teilweise zu ersetzen, in dem die abgetrennte Flüssigkeit in eine Alkohol- und Wasserphase getrennt wird und die Alkoholphase in die Veresterungsreaktion zurückgeführt wird.

Optional kann frischer Alkohol zur abgretrennten Alkoholphase beigefügt werden.

Für die Zufuhr des Alkohols in die Veresterungsreaktion gibt es verschiedene Möglichkeiten. Der Alkohol kann beispielsweise als Rückfluss auf die Kolonne gegeben werden. Eine andere Möglichkeit ist, den Alkohol, gegebenenfalls nach Aufheizung, in das flüssige Reaktionsgemisch zu pumpen. Durch die Abtrennung des Reaktionswasser sinkt das Reaktionsvolumen in der Apparatur. Im Idealfall wird während der Reaktion so viel Alkohol nachgespeist, wie dem Volumen des abgetrennten Destillats (Wasser und gegebenenfalls Alkohol) entspricht, so dass der Füllstand im Reaktionsgefäß konstant bleibt. Im erfindungsgemäßen Verfahren wird durch die Vergrößerung des Alkoholüberschusses das Gleichgewicht zu Gunsten der Vollester verschoben.

Nach Beendigung der Reaktion enthält das Reaktionsgemisch, das im Wesentlichen aus Vollester (Zielprodukt) und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird der überschüssige Alkohol entfernt, die saueren Verbindungen neutralisiert, der Katalysator zerstört und die dabei entstandenen festen Nebenprodukte abgetrennt. Dabei wird der größte Teil des Alkohols bei Normaldruck oder im Vakuum abdestilliert. Die letzten Spuren des Alkohols können z. B. durch Wasserdampfdestillation, inbesondere im Temperaturbereich von 120 bis 225 °C, entfernt werden. Die Abtrennung des Alkohols kann als erster oder als letzter Aufarbeitungsschritt erfolgen.

Die Neutralisation der saueren Stoffe, wie Carbonsäuren, Estercarbonsäuren oder gegebenfalls der saueren Katalysatoren, erfolgt durch Zugabe von basisch wirkenden Verbindungen der Alkali- und Erdalkalimetalle. Diese werden in Form ihrer Carbonate, Hydrogencarbonate oder Hydroxide eingesetzt. Das Neutralisationsmittel kann in fester Form oder bevorzugt als Lösung, insbesondere als wässrige Lösung eingesetzt werden. Hier wird häufig Natronlauge im Konzentrationsbereich von 1 bis 30 Gew.-%, bevorzugt von 20 bis 30 Gew.-% verwendet. Das Neutralisationsmittel wird in einer Menge zugesetzt, die dem Einfachen bis dem Vierfachen, insbesondere dem Einfachen bis Zweifachen der stöchiometrisch notwendigen Menge, die durch Titration bestimmt wird, entspricht. Durch das Neutralisationsmittel werden bei Verwendung von Titankatalysatoren diese in feste, filtrierbare Stoffe umgewandelt.

Die Neutralisation kann sofort nach Beendigung der Veresterungsreaktion oder nach Abdestillation der Hauptmenge des überschüssigen Alkohols durchgeführt werden. Bevorzugt ist die Neutralisation mit Natronlauge sofort nach Beendigung der Veresterungsreaktion bei Temperaturen über 150 °C. Das mit der Lauge eingebrachte Wasser kann dann zusammen mit Alkohol abdestilliert werden.

Die im neutralisierten Rohester enthaltenden Feststoffe können durch Zentrifugieren oder bevorzugt durch Filtration abgetrennt werden.

Optional kann nach der Veresterungsreaktion während der Aufarbeitung zur besseren Filtrierbarkeit und/oder Abtrennung von farbigen Substanzen oder sonstigen Nebenprodukten ein Filterhilfsmittel und/oder Absorptionsmittel zugesetzt werden.

Das hier beschriebene Verfahren kann in einem Behälter oder in mehreren hintereinander geschalteten Gefäßen durchgeführt werden. So kann beispielsweise die Veresterung und die Aufarbeitung in verschiedenen Gefäßen erfolgen. Wenn Carbonsäureanhydride eingesetzt werden, besteht die Option, die Reaktionen zum Halbester und Diester in verschiedenen Reaktoren durchzuführen.

Die so hergestellten Ester aus mehrbasischen Carbonsäuren, wie beispielsweise Phthalsäure, Adipinsäure, Sebacinsäure, Maleinsäure, und aus Alkoholen, finden weite Anwendung in Lackharzen, als Bestandteile von Anstrichmitteln und insbesondere als Weichmacher für Kunststoffe. Geeignete Weichmacher für PVC sind Diisononylphthalate und Dioctylphthalate. Die Verwendung der erfindungsgemäß hergestellten Ester für diese Zwecke ist ebenfalls Gegenstand der Erfindung.

Der bei der Aufarbeitung abgetrennte Alkohol kann, gegebenenfalls nach Ausschleusung einer Teilmenge, für den nächsten Ansatz verwendet werden.

Die folgenden Beispiele sollen die Erfindung näher erläutern, ohne den Schutzbereich, wie in den Patentansprüchen definiert, zu beschränken.

### Beispiele

Der verwendete Veresterungsreaktor besteht aus einem Rührbehälter mit Heizschlange (40 bar Dampf), einem Trennsystem für die Reaktionswasser/Alkohol-Trennung und einer Rücklaufleitung für den Überschussalkohol. Die Apparatur wird vor Befüllung mit Stickstoff sauerstofffrei gespült.

### Veresterung von Phthalsäureanhydrid mit einem Gemisch isomerer Isononanole zu Diisononylphthalat (DINP)

### Beispiel 1 (Vergleichsbeispiel)

### Einsatzmengen:

1000 kg Phthalsäureanhydrid (flüssig)
2430 kg Isononanol
1 kg Butyltitanat

Sobald 400 kg Isononanol im Reaktor vorgelegt worden waren, wurde mit dem Aufheizen begonnen. Phthalsäureanhydrid in flüssiger Form und die Restalkoholmenge (2.030 kg) wurden gleichzeitig eingespeist. Nachdem das Reaktionsgemisch eine Temperatur von 120 °C erreicht hatte, wurde der Titankatalysator zugegeben. Bei 170 °C begann das Gemisch zu sieden. Zu diesem Zeitpunkt stellte sich auch der maximale Füllstand mit 80 % im Reaktor ein. Während der Veresterung wurde Wasser freigesetzt und als Isononanol-Azeotrop abdestilliert. Die Säurezahl der Reaktionsmischung sank von einem Startwert von 100 mg KOH/g bei Siedebeginn auf 10 mg KOH/g nach 150 Minuten, 1 mg KOH nach 290 Minuten und 0,5 mg KOH/g nach 330 Minuten. Der Stand im Reaktor betrug zu diesem Zeitpunkt noch 76 %.

### Beispiel 2 (gemäß der Erfindung)

### Einsatzmengen:

1000 kg Phthalsäureanhydrid (flüssig)
2430 kg Isononanol
110 kg Isononanol (Nachdosierung)
1 kg Butyltitanat

Sobald 400 kg Isononanol im Reaktor vorgelegt worden waren, wurde mit dem Aufheizen begonnen. Phthalsäureanhydrid in flüssiger Form und die Restalkoholmenge (2.030 kg) wurden gleichzeitig eingespeist. Nachdem das Reaktionsgemisch eine Temperatur von 120 °C erreicht hatte, wurde der Titankatalysator zugegeben. Bei 170 °C begann das Gemisch zu sieden. Zu diesem Zeitpunkt stellte sich auch der maximale Füllstand mit 80 % im Reaktor ein. Während der Veresterung wurde Wasser freigesetzt und als Isononanol-Azeotrop abdestilliert. Bei einem Stand von 78 % im Reaktor (ca. 2 h nach Siedebeginn) wurde dieser mit Frischisononanol (nicht in der Einsatzmenge enthalten) wieder auf 80 % gebracht und dieser Füllstand durch weitere Nachdosierung von Isononanol bis zum Ende der Reaktion gehalten. Bei der Beendigung der Reaktion (SZ = 0,5) war der Isononanolüberschuss um 110 kg höher als im Vergleichsbeispiel 1. Die Säurezahl der Reaktionsmischung sank bei diesem Ansatz von einem Startwert von 100 mg KOH/g bei Siedebeginn auf 10 mg KOH/g nach 150 Minuten, 1 mg KOH nach 270 Minuten und 0,5 mg KOH/g nach 300 Minuten.

Nach dem erfindungsgemäßen Verfahren wird demnach die Veresterungszeit um 30 Minuten oder um 9 % verkürzt. (Ausbeute bezogen auf Phthalsäureanhydrid größer 99,8 %)

### Veresterung von Phthalsäureanhydrid mit 2-Ethylhexanol zu Bis(2-ethylhetyl)phthalat (DOP)

### Beispiel 3 (Vergleichsbeispiel)

### Einsatzmengen:

1070 kg Phthalsäureanhydrid (flüssig)
2350 kg 2-Ethylhexanol
1 kg Butyltitanat

Sobald 400 kg 2-Ethylhexanol im Reaktor vorgelegt worden waren, wurde mit dem Aufheizen begonnen. Phthalsäureanhydrid in flüssiger Form und die Restalkoholmenge (1.950 kg) wurden gleichzeitig eingespeist. Nachdem das Reaktionsgemisch eine Temperatur von 120 °C erreicht hatte, wurde der Titankatalysator zugegeben. Bei 170 °C begann das Gemisch zu sieden. Zu diesem Zeitpunkt stellte sich auch der maximale Füllstand mit 80 % im Reaktor ein. Während der Veresterung wurde Wasser freigesetzt und als 2-Ethylhexanol-Azeotrop abdestilliert. Die Säurezahl der Reaktionsmischung sank von einem Startwert von 110 mg KOH/g bei Siedebeginn auf 10 mg KOH/g nach 165 Minuten, 1 mg KOH nach 320 Minuten und 0,5 mg KOH/g nach 365 Minuten. Der Stand im Reaktor betrug zu diesem Zeitpunkt noch 76 %.

### Beispiel 4 (gemäß der Erfindung)

### Einsatzmengen:

1070 kg Phthalsäureanhydrid (flüssig)
2350 kg 2-Ethylhexanol
1 kg Butyltitanat

Sobald 400 kg 2-Ethylhexanol im Reaktor vorgelegt worden waren, wurde mit dem Aufheizen begonnen. Phthalsäureanhydrid in flüssiger Form und die Restalkoholmenge (1.950 kg) wurden gleichzeitig eingespeist. Nachdem das Reaktionsgemisch eine Temperatur von 120 °C erreicht hatte, wurde der Titankatalysator zugegeben. Bei 170 °C begann das Gemisch zu sieden. Zu diesem Zeitpunkt stellte sich auch der maximale Füllstand mit 80 % im Reaktor ein. Während der Veresterung wurde Wasser freigesetzt und als 2-Ethylhexanol-Azeotrop abdestilliert. Bei einem Stand von 78 % im Reaktor (ca. 2 h nach Siedebeginn) wurde dieser mit Frisch-2-Ethylhexanol (nicht in der Einsatzmenge enthalten) wieder auf 80 % gebracht und dieser durch weitere Nachdosierung von 2-Ethylhexanol bis zum Ende der Reaktion gehalten. Bei der Beendigung der Reaktion (SZ = 0,5) war der 2-Ethylhexanolüberschuss um 120 kg höher als im Vergleichsbeispiel 3. Die Säurezahl der Reaktionsmischung sank bei diesem Ansatz von einem Startwert von 110 mg KOH/g bei Siedebeginn auf 10 mg KOH/g nach 165 Minuten, 1 mg KOH/g nach 300 Minuten und 0,5 mg KOH/g nach 325 Minuten.

Dieses Beispielpaar zeigt, dass nach dem erfindungsgemäßen Verfahren die Veresterungszeit um 40 Minuten oder 11 % verkürzt wird (Ausbeute größer als 99,8 % bezogen auf Phthalsäureanhydrid).

## Patentansprüche

1. Verfahren zur Herstellung von Carbonsäureestern durch Reaktion von Di- oder Polycarbonsäuren oder deren Anhydride mit Alkoholen, wobei das Reaktionswasser durch azeotrope Destillation mit dem Alkohol entfernt wird,
**dadurch gekennzeichnet,**
**dass** die durch die azeotrope Destillation aus der Reaktion entfernte Flüssigkeitsmenge vollständig mit dem Alkohol wieder ergänzt wird.

2. Verfahren nach Anspruch 1,
**dadurch gekennzeichnet,**
**dass** die durch azeotrope Destillation abgetrennte Flüssigkeitsmenge vollständig ergänzt wird, indem die abgetrennte Flüssigkeit in eine Wasserphase und eine Alkoholphase getrennt wird und die Alkoholphase, zusätzlich mit frischem Alkohol versetzt, in die Veresterungsreaktion zurückgeführt wird.

3. Verfahren nach Anspruch 1,
**dadurch gekennzeichnet,**
**dass** die durch die azeotrope Destillation aus der Reaktion entfernte Flüssigkeitsmenge durch frischen Alkohol vollständig wieder ergänzt wird.

4. Verfahren nach Anspruch 1 bis 3,
**dadurch gekennzeichnet,**
**dass** als Di- oder Carbonsäure Phthalsäure oder Phthalsäureanhydrid eingesetzt wird.

5. Verfahren nach Anspruch 1 bis 3,
**dadurch gekennzeichnet,**
**dass** als Alkohol n-Butanol, Isobutanol, n-Octanol (1), n-Octanol (2), 2-Ethylhexanol, Nonanole, Decylalkohole oder Tridecanole eingesetzt werden.

## Claims

1. A process for preparing a carboxylic ester by reacting a dicarboxylic or polycarboxylic acid or its anhydride with an alcohol, the reaction water being removed by azeotropic distillation together with the alcohol, **characterized in that** the amount of liquid removed from the reaction by the azeotropic distillation is replaced wholly with the alcohol.

2. A process according to claim 1, **characterized in that** the amount of liquid separated off by the azeotropic distillation is supplemented wholly by separating the liquid separated off into a water phase and an alcohol phase and recirculating the alcohol phase, additionally admixed with fresh alcohol, to the esterification reaction.

3. A process according to claim 1, **characterized in that** the amount of liquid removed from the reaction by the azeotropic distillation is replaced wholly by fresh alcohol.

4. A process according to any of claims 1 to 3, **characterized in that** the dicarboxylic or carboxylic acid used is phthalic acid or phthalic anhydride.

5. A process according to any of claims 1 to 3, **characterized in that** the alcohol used is n-butanol, isobutanol, n-octanol (1), n-octanol (2), 2-ethylhexanol, nonanols, decyl alcohols or tridecanols.

## Revendications

1. Procédé de préparation d'esters d'acides carboxyliques par réaction d'acides dicarboxyliques ou polycarboxyliques ou de leurs anhydrides avec des alcools, l'eau de réaction étant éliminée par distillation azéotrope avec l'alcool,
**caractérisé en ce que**
la quantité de liquide éliminée de la réaction par distillation azéotrope est entièrement complétée avec l'alcool.

2. Procédé selon la revendication 1,
**caractérisé en ce qu'**
on complète entièrement la quantité de liquide séparée par distillation azéotrope en séparant le liquide séparé en une phase eau et une phase alcool et en recyclant la phase alcool, allongée en complément avec de l'alcool frais, dans la réaction d'estérification.

3. Procédé selon la revendication 1,
**caractérisé en ce qu'**
on complète entièrement la quantité de liquide éliminée de la réaction par distillation azéotrope avec de l'alcool frais.

4. Procédé selon les revendications 1 à 3,
**caractérisé en ce que**
comme acide dicarboxylique ou carboxylique, on utilise l'acide phtalique ou l'anhydride de l'acide phtalique.

5. Procédé selon les revendications 1 à 3,
**caractérisé en ce que**
comme alcool, on utilise du n-butanol, de l'isobutanol, du n-octanol (1), du n-octanol (2), du 2-éthylhexanol, des nonanols, des alcools décyliques ou des tridécanols.
